# EUROPÄISCHE PATENTSCHRIFT

(11) **EP 1 008 573 B1**
(45) Veröffentlichungstag und Bekanntmachung des Hinweises auf die Patenterteilung: **12.06.2002**
(21) Anmeldenummer: 99122885.9
(22) Anmeldetag: 18.11.1999
(51) Int. Cl.: C07C 5/05

(54) **Verfahren zur Hydrierung von Polyenen zu Monoenen**
Process for the hydrogenation of polyenes to monoenes
Procédé pour l'hydrogénation de polyènes en monoènes

(30) Priorität: 09.12.1998 DE 19856862
(43) Veröffentlichungstag der Anmeldung: 14.06.2000
(73) Patentinhaber: BASF AKTIENGESELLSCHAFT, 67056 Ludwigshafen (DE)
(72) Erfinder: Pinkos, Rolf, Dr., 67098 Bad Dürkheim (DE)

(56) Entgegenhaltungen:
- US-A- 5 128 296
- US-A- 5 177 278

## Beschreibung

Die vorliegende Patentanmeldung betrifft ein Verfahren zur Hydrierung von Polyenen zu Monoenen mit homogenen Rutheniumkatalysatoren in Gegenwart von Carbonsäuren.

Ein Haupteinsatzgebiet für das erfindungsgemäße Verfahren ist die Hydrierung von 1,5,9-Cyclododecatrien (CDT) zu Cyclododecen (CDE). CDE findet Verwendung, z.B. als Zwischenprodukt zur Herstellung von Polyamid über 1,12-Dodecan-disäure, 1,12-Diaminododecan und/oder dem Lactam der 12-Amino-dodecansäure. Ferner kann es als Edukt zur Herstellung von Riechstoffen wie 2-Cyclododecyl-1-propanol, einem begehrten Moschusriechstoff, dienen.

Bei der Hydrierung von Polyenen zu den entsprechenden Monoenen ist es im allgemeinen sehr wichtig, daß die Ausbeuten bei diesen Reaktionen sehr hoch sind, da sich aufgrund der geringen Massenund Polaritätsunterschiede zwischen Edukten und Reaktionsprodukten, diese sich nicht oder nur sehr aufwendig destillativ voneinander trennen lassen. D.h. der Polyenumsatz muß möglichst quantitativ sein. Üblicherweise werden Ausbeuten von über 98 % angestrebt.

In US 5 321 176 z.B. wird die Hydrierung von Polyenen zu Monoenen an homogenen Ru-Katalysatoren unter Zusatz von Wasser beschrieben. In Beispiel 2 wird unter Zusatz von Wasser nach 4 Stunden (h) Reaktionszeit ein CDT-Umsatz von 98,4 % erreicht. Welche CDE-Ausbeute erhalten wird, ist nicht beschrieben. In Beispiel 1 wird unter Zusatz von etwas weniger Wasser nach 8 h Reaktionszeit nur ein unbefriedigender Umsatz von 85,8 % erreicht. Damit scheint die Zeit, die für einen quantitativen Umsatz notwendig ist, je nach Versuchsbedingungen stark zu schwanken, wodurch dieses Verfahren technisch nicht leicht durchführbar ist, da es nicht ermöglicht, bei quantitativem Umsatz immer hohe CDE-Ausbeuten zu erhalten.

In US 5 180 870 ist ein Verfahren zur Hydrierung von Polyenen mit homogen Ruthenium-Katalysatoren unter Zusatz von Aminen zum Reaktionsgemisch beschrieben. Aus keinem Patentbeispiel wird ersichtlich, wie man bei quantitativem Umsatz hohe CDE-Ausbeuten erhalten soll. Darüber hinaus ist bei Zusatz von Aminen immer zu befürchten, daß der Produktstrom mit Aminen verunreinigt bleibt. Dies ist vor allem bei der Produktion von Riechstoffen aufgrund der den meisten Aminen eigenen intensiven Gerüche als sehr problematisch anzusehen.

Gemäß dem Verfahren von US 5 177 278 wird die CDT-Hydrierung mit homogenen Ru-Katalysatoren in Gegenwart von Ethern oder Estern mit einem Siedepunkt höher als 245°C als Lösungsmittel vorgenommen. Nach den Patentbeispielen liegen die besten CDE-Selektivitäten bei 96 bis 98 %, dies allerdings in keinem Fall bei quantitativem Umsatz, so daß sich bei der Aufarbeitung des Reaktionsgemisches große Trennprobleme ergeben. Ferner verringern sich bei der Hydrierung in Gegenwart von großen Lösungsmittelmengen die Raum-Zeit-Ausbeuten, da durch die Verdünnung wertvoller Reaktionsraum verloren geht. Dieser nachteilige Effekt tritt ebenfalls bei den Verfahren gemäß den Patenten US 3 804 914, US 5 210 349 und US 5 128 296 auf, obwohl hierbei teilweise recht hohe CDE-Ausbeuten erzielt werden.

Gemäß dem Verfahren der veröffentlichten Patentanmeldung US B 316,917 wird ebenfalls in Lösungsmitteln gearbeitet, die maximale CDE-Ausbeute ist mit ca. 95 % beschrieben. Allerdings ist der Umsatz nicht vollständig.

Von D.R. Fahey, wird im J. Org. Chem. 38, (1973) Seiten 80-87 die Hydrierung von CDT an verschiedenen homogenen Ru-Katalysatoren ausführlich beschrieben. Auch hierin wird in allen Beispielen in Gegenwart größerer Mengen an Lösungsmitteln gearbeitet. Es werden CDE-Ausbeuten von ca. 98 % beschrieben. Allerdings ist die beschriebene Einsatzmenge an Ru, bezogen auf CDT, sehr hoch. Umgerechnet auf 1 kg CDT bzw. CDE werden nämlich 6 bis 7 g Ruthenium (berechnet als Metall) eingesetzt. Berücksichtigt man, daß 1 g Ruthenium ca. 2,- DM kostet, ergeben sich bei diesem Verfahren allein Ru-Kosten/kg Produkt von circa 12,- bis 14,- DM. Daher ergibt sich nur ein in industriellem Maßstab wirtschaftliches Verfahren, wenn der Katalysator sehr oft zurückgeführt werden kann. Selbst beim Arbeiten mit Rückführung des Ruthenium-KatalySators ergeben sich jedoch üblicherweise Probleme, wie Veränderungen des Katalysators, die dann zu Einbußen in der Aktivität des Katalysators und damit in der Selektivität führen. So ist z.B. mit der Oxidation des Katalysators durch Sauerstoffspuren zu rechnen.

Es war daher die Aufgabe der Erfindung ein Verfahren zu entwickeln, mit dem man Polyene unter Vermeidung der Nachteile der Verfahren des Standes der Technik mit homogenen Ru-Katalysatoren zu den entsprechenden Monoenen hydrieren kann. Dies bedeutet also, daß man das Verfahren auch ohne Verwendung von großen Mengen eines teuren Katalysators und möglichst ohne Verwendung eines Lösungsmittels oder zumindest mit wenig Lösungsmittel vorteilhaft durchführen kann und trotzdem reproduzierbar bei quantitativem Umsatz eine sehr hohe Ausbeute erreicht.

Es wurde nun überraschend gefunden, daß sich Polyene mit homogenen Ru-Katalysatoren bei vollständigem Umsatz und hohen Ausbeuten zu den entsprechenden Monoenen hydrieren lassen, wenn man die Hydrierung in Gegenwart von Carbonsäuren durchführt.

Gegenstand der Erfindung ist dementsprechend ein Verfahren zur Hydrierung von Polyenen mit homogenen Ru-Katalysatoren zu den entsprechenden Monoenen, das dadurch gekennzeichnet ist, daß man die Hydrierung in Gegenwart einer Carbonsäure durchführt.

Es war sehr überraschend, daß der Zusatz der Carbonsäuren, eine merkliche Weiterhydrierung des Cyclododecens verhindert.

Als Polyene kommen z.B. cyclische Polyene wie 1,5,9-Cyclododecatrien, 2,4-Dimethyl-cyclododecatrien, 4-n-Butyl-cyclododecatrien, 1-Cyclohexyl-dodecatrien, 1-Phenyl-cyclododecatrien, 1,5-Cyclooctadien, Cyclooctatetraen, Bicyclo[2.2.1]hepta-2,5-dien und Bicyclo[2.2.2]octa-2,5-dien in Betracht. Bevorzugt sind 1,5,9-Cyclododecatrien und 1,5-Cyclooctadien, insbesondere 1,5,9-Cyclododecatrien.

Als Ru-Katalysatoren können beispielsweise solche eingesetzt werden, wie sie in den oben erläuterten Patentschriften US 5 180 870, US 5 321 179, US 5 177 278, US 3 804 914, US 5 210 349, US 5 128 296, US B 316 917 bzw. in dem Artikel von D.R. Fahey in J. Org. Chem. 38, (1973) Seiten 80-87, beschrieben sind. Als bevorzugte Katalysatoren werden (TPP)₂(CO)₂RuCl₂ oder die entsprechenden Chlor-freien Varianten eingesetzt. Hierin bedeutet TPP Triphenylphosphin.

Da in einer bevorzugten Ausführungsform der Erfindung der Katalysator in situ hergestellt wird, ist anzunehmen, daß Gemische von Ru-Verbindungen vorliegen. Dabei dürften aktive Katalysatorkomplexe in der Hydridform vorliegen. Sofern der Katalysator in situ hergestellt wird, geht man z.B. von Rutheniumchlorid, Rutheniumacetat, Rutheniumacetylacetonat oder anderen käuflich erhältlichen Ru-Verbindungen aus.

Üblicherweise wird dem Reaktionsgemisch außer einer Ru-Komponente zusätzlich NR₃, PR₃, AsR₃ oder SbR₃ zugesetzt, worin R eine Alkyl- und/oder eine Arylgruppe bedeutet. Bevorzugt verwendet man zusätzlich Triphenylphosphin.

Bezogen auf 1 kg Polyen wird im erfindungsgemäßen Verfahren Ru zwischen 0,1 und 2000 mg, vorzugsweise zwischen 1 und 1000 mg, insbesondere 10 bis 200 mg (berechnet als Metall) eingesetzt. Hierdurch ergeben sich so niedrige Katalysatorkosten, daß der Katalysator nicht unbedingt zurückgeführt werden muß. Selbstverständlich kann dies aber auch durchgeführt werden, wobei sich der Carbonsäurezusatz stabilisierend auf den Katalysator auswirkt und dieser bei wiederholtem Einsatz aktiv und selektiv wie zuvor ist.

Als Carbonsäuren können beispielsweise aliphatische, cycloaliphatische, aromatische oder araliphatische Carbonsäuren verwendet werden. Bevorzugt werden solche eingesetzt, die unter den Reaktionsbedingungen im Reaktionssystem löslich sind. Genannt seien beispielsweise C₁- bis C₂₀-Monocarbonsäuren, C₂- bis C₆-Dicarbonsäuren, Cyclohexylcarbonsäure, Benzoesäure, Terephthalsäure, Phthalsäure und Phenylessigsäure. Besonders bevorzugte Säuren sind aliphatische Mono- und Dicarbonsäuren, insbesondere Essigsäure, Propionsäure sowie C₁₂ bis C₂₀-Fettsäuren, Bernsteinsäure und Adipinsäure.

Die Menge der zugesetzten Carbonsäure pro kg Polyen liegt im allgemeinen zwischen 0,001 und 100 g, vorzugsweise zwischen 0,01 und 50 g, insbesondere bei 0,05 bis 25 g.

Bei der in situ Herstellung des Katalysators wird noch eine CO-Quelle zugesetzt. Diese kann entweder gasförmiges CO selbst sein, oder aber auch Formaldehyd.

Das erfindungsgemäße Verfahren wird im allgemeinen bei Temperaturen von 50 bis 300°C, vorzugsweise von 80 bis 250°C und insbesondere zwischen 100 und 200°C ausgeübt. Die Reaktionsdrücke liegen dabei im allgemeinen zwischen 1 und 300 bar, vorzugsweise zwischen 1 und 200 bar, insbesondere zwischen 1 und 100 bar.

Die Reaktionszeiten pro Ansatz, bzw. bei kontinuierlicher Verfahrensführung die Verweilzeiten, liegen im allgemeinen zwischen 0,5 und 48 Stunden. Sie richten sich im wesentlichen nach den Ansatzgrößen und den Möglichkeiten Energie zu- bzw. abführen zu können. Durch die Anwesenheit von Carbonsäuren im Reaktionsgemisch ist es nicht kritisch, wenn der Reaktionsansatz länger als erforderlich unter den Reaktionsbedingungen gehandhabt wird. Dadurch ist eine erheblich vereinfachte Versuchsführung und Versuchskontrolle möglich.

Die nachstehenden Beispiele sollen das erfindungsgemäße Verfahren näher erläutern.

### Beispiel 1

In einen 2,5-1-Rührautoklaven wurden 1 kg trans-, trans-, cis-1,5,9-Cyclododecatrien (Firma Sigma-Aldrich, D-89555 Steinheim), 150 mg RuCl₃*H₂O, 20 g Triphenylphosphin, 12,5 g einer 37%igen wäßrigen Formaldehydlösung, 25 ml Ethanol und 2,5 g Essigsäure eingefüllt. Nach dem Spülen des Reaktors mit Stickstoff bzw. Wasserstoff wurden 15 bar Wasserstoff aufgepreßt. Danach wurde der Reaktor unter Rühren aufgeheizt. Bei einer Temperatur von ca. 130°C nahm der Reaktordruck merklich ab und es setzte eine exotherme Reaktion ein, so daß die Temperatur bis auf 163°C anstieg. Die Temperatur wurde anschließend zwischen 140 und 150°C gehalten, indem portionsweise Wasserstoff nachgepreßt wurde. Der maximale Druck betrug dabei 20 bar. Nach beendeter Wasserstoffaufnahme fanden sich im Reaktionsaustrag laut gaschromatographischer Analyse (GC) 98,1 % Cyclododecen und 1,8 % Cyclododecan. Die Ausbeute betrug somit 98,2 % der Theorie.

### Beispiel 2

In einen 70-ml-Autoklav mit Magnetrührer wurden 40 g trans-, trans-, cis-1,5,9-Cyclododeca-trien, 50 mg RuCl₃*H₂O, 400 mg Triphenylphosphin, 0,5 g einer 37%igen wäßrigen Formaldehydlösung und 100 mg Essigsäure vorgelegt. Nach Spülen des Reaktors mit Stickstoff und Wasserstoff wurde der Reaktor auf 20 bar Wasserstoffdruck gebracht und unter Rühren aufgeheizt. Bei einer Temperatur von 115°C sank der Reaktordruck merklich ab und die Innentemperatur stieg innerhalb kurzer Zeit auf 140°C. Danach wurde der Reaktor mittels Wasserstoff ständig bei 20 bar und einer Temperatur von 145°C gehalten. Nach etwa. 2,5 Stunden wurde abgekühlt und entspannt. Im Austrag wurde durch GC eine Ausbeute von 97 % Cyclododecen und 1,8 % Cyclododecan festgestellt. Der Rest war Cyclododecadien.

### Vergleichsbeispiel

Arbeitete man wie in Beispiel 2 beschrieben, jedoch ohne Zusatz von Essigsäure so wurde im Austrag durch GC eine Ausbeute von nur 86,8 % Cyclododecen und von 13,2 % Cyclododecan festgestellt.

## Patentansprüche

1. Verfahren zur Hydrierung von Polyenen mit homogenen Ru-Katalysatoren zu den entsprechenden Monoenen, **dadurch gekennzeichnet, daß** man die Hydrierung in Gegenwart einer Carbonsäure durchführt.

2. Verfahren gemäß Anspruch 1, **dadurch gekennzeichnet, daß** man den homogenen Ru-Katalysator vor der Hydrierung in situ erzeugt.

3. Verfahren gemäß Anspruch 1, **dadurch gekennzeichnet, daß** man den Katalysator vor der Hydrierung in situ in Gegenwart von CO oder Formaldehyd erzeugt.

4. Verfahren gemäß Anspruch 1, **dadurch gekennzeichnet, daß** die Hydrierung in Gegenwart von Triphenylphosphin durchgeführt wird.

5. Verfahren gemäß Anspruch 1, **dadurch gekennzeichnet, daß** die Hydrierung bei Temperaturen von 50 bis 300°C und Drucken von 1 bis 300 bar durchgeführt wird.

6. Verfahren gemäß Anspruch 1, **dadurch gekennzeichnet, daß** man als Polyen 1,5,9-Cyclododecatrien verwendet.

7. Verfahren gemäß Anspruch 1, **dadurch gekennzeichnet, daß** man als Polyen 1,5-Cyclooctadien verwendet.

8. Verfahren gemäß Anspruch 1, **dadurch gekennzeichnet, daß** man pro kg Polyen zwischen 0,1 und 2000 mg Ru (berechnet als Metall) einsetzt.

9. Verfahren gemäß Anspruch 1, **dadurch gekennzeichnet, daß** man als Carbonsäure eine C₁- bis C₂₀-Monocarbonsäure, eine C₂bis C₆-Dicarbonsäure, Cyclohexylcarbonsäure, Benzoesäure, Terephthalsäure, Phthalsäure oder Phenylessigsäure verwendet.

10. Verfahren gemäß Anspruch 1, **dadurch gekennzeichnet, daß** man als Carbonsäure Essigsäure, Propionsäure, Bernsteinsäure oder Adipinsäure oder eine C₁₂-bis C₂₀-Fettsäure verwendet.

## Claims

1. A process for hydrogenating polyenes to the corresponding monoenes using homogeneous ruthenium catalysts, which comprises effecting said hydrogenation in the presence of a carboxylic acid.

2. A process as claimed in claim 1, wherein the homogeneous ruthenium catalyst is generated in situ prior to said hydrogenation.

3. A process as claimed in claim 1, wherein the catalyst is generated in situ in the presence of CO or formaldehyde prior to said hydrogenation.

4. A process as claimed in claim 1, wherein said hydrogenation is effected in the presence of triphenylphosphine.

5. A process as claimed in claim 1, wherein said hydrogenation is effected at from 50 to 300°C and at from 1 to 300 bar.

6. A process as claimed in claim 1, wherein the polyene used is 1,5,9-cyclododecatriene.

7. A process as claimed in claim 1, wherein the polyene used is 1,5-cyclooctadiene.

8. A process as claimed in claim 1, wherein from 0.1 to 2000 mg of ruthenium (reckoned as metal) is used per kg of polyene.

9. A process as claimed in claim 1, wherein the carboxylic acid used is a C₁-C₂₀ monocarboxylic acid, a C₂-C₆ dicarboxylic acid, cyclohexylcarboxylic acid, benzoic acid, terephthalic acid, phthalic acid or phenylacetic acid.

10. A process as claimed in claim 1, wherein the carboxylic acid used is acetic acid, propionic acid, succinic acid or adipic acid or a C₁₂-C₂₀ fatty acid.

## Revendications

1. Procédé pour l'hydrogénation de polyènes avec des catalyseurs au Ru homogènes pour donner les monoènes correspondants, **caractérisé par le fait qu'**on effectue l'hydrogénation en présence d'un acide carboxylique.

2. Procédé selon la revendication 1, **caractérisé par le fait qu'**on produit le catalyseur au Ru homogène in situ avant l'hydrogénation.

3. Procédé selon la revendication 1, **caractérisé par le fait qu'**on produit le catalyseur in situ avant l'hydrogénation, en présence de CO ou de formaldéhyde.

4. Procédé selon la revendication 1, **caractérisé par le fait qu'**on effectue l'hydrogénation en présence de triphénylphosphine.

5. Procédé selon la revendication 1, **caractérisé par le fait qu'**on effectue l'hydrogénation à des températures de 50à 300°C et des pressions de 1 à 300 bar.

6. Procédé selon la revendication 1, **caractérisé par le fait qu'**on utilise comme polyène le 1,5,9-cyclododécatriène.

7. Procédé selon la revendication 1, **caractérisé par le fait qu'**on utilise comme polyène le 1,5-cyclooctatriène.

8. Procédé selon la revendication 1, **caractérisé par le fait qu'**on utilise par kg de polyène entre 0,1 et 2000 mg de Ru (calculé en tant que métal).

9. Procédé selon la revendication 1, **caractérisé par le fait qu'**on utilise comme acide carboxylique un acide monocarboxylique en C₁ à C₂₀, un acide dicarboxylique en C₂ à C₆, l'acide cyclohexylcarboxylique, l'acide benzoïque, l'acide téréphtalique, l'acide phtalique ou l'acide phénylacétique.

10. Procédé selon la revendication 1, **caractérisé par le fait qu'**on utilise comme acide carboxylique l'acide acétique, l'acide propionique, l'acide succinique ou l'acide adipique ou un acide gras en C₁₂ à C₂₀.
